(19) Europäisches Patentamt
European Patent Office
Office européen des brevets

(11) Publication number : **0 338 173 B1**

# (12) EUROPEAN PATENT SPECIFICATION

(45) Date of publication of patent specification : **26.02.92 Bulletin 92/09**

(51) Int. Cl.$^5$ : **A61L 15/16**

(21) Application number : **88402820.0**

(22) Date of filing : **09.11.88**

## (54) Ionic dressing for topical administration of drugs to wounds and burns.

(30) Priority : **22.04.88 CA 564950**

(43) Date of publication of application : **25.10.89 Bulletin 89/43**

(45) Publication of the grant of the patent : **26.02.92 Bulletin 92/09**

(84) Designated Contracting States : **AT BE CH DE ES FR GB GR IT LI LU NL SE**

(56) References cited :
**GB-A- 2 007 096**
**US-A- 3 817 702**
**US-A- 4 585 652**

(73) Proprietor : **RICOH KYOSAN, INC.**
**Yaesu-Mitsui Bldg. 7-2 Yaesu 2-Chome**
**Chuo-ku**
**Tokyo (JP)**

(72) Inventor : **Yamazaki, Hiroshi**
**22 Alderbrook Drive**
**Nepean Ontario, K2H 5W5 (CA)**
Inventor : **Miyazaki, Masao**
**6-4-111,1-Chome Hikarigaoka**
**Nerima-ku Tokyo (JP)**

(74) Representative : **Brycman, Jean et al**
**CABINET ORES 6, Avenue de Messine**
**F-75008 Paris (FR)**

EP 0 338 173 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to ionic forms of dressings to which a variety of ionic, physiologically- and/or biologically-active materials can be adsorbed so that a controlled release of that material into body exudates of wounds can take place.

When drugs are systemically administered to treat wounds (including cuts, abrasions, incisions, ulcers and infected wounds or burns), a large portion of the drugs is either degraded or adsorbed by nontarget tissues and only a small portion of the initial dose reaches the target site. The efficiency of systemic dosing is further decreased in the trauma patient (as in accidents, earthquakes, fires and wars) who often suffer a decreased vascular flow and thus have reduced drug circulation. Furthermore, the trauma patient often fails to provide information regarding sensitivity to drugs, or fails to take drugs orally. Topical drug administration, in theory, would provide immediate, direct, and sustained effects at the target site, and reduce side-effects and degradation of drugs encountered in systemic dosing. Topical application also permits rapid removal and replacement of drugs when adverse effects are noticed. When cleansing is not readily available, topical application is more effective in destroying microbial spores because a higher concentration of drugs can be applied. Thus, treatment of wounds or burns will benefit from an improvement of topical administration, whether used alone or in conjunction with systemic dosing.

Currently, antibiotics, e.g. fusidic acid, chlorohexidine, Neomycin, Polymyxin and Bacitracin are topically applied in gel, cream or ointment forms (occasionally in aerosol and powder). Because a high concentration of the drugs are in direct contact with the target tissue, some of the drugs cause allergic reaction by contact with the target tissue, some of drugs cause allergic dermatitis, particularly in patients with stasis ulcers or eczema, or exhibit toxicity.

To provide a controlled release of drugs, a U.S. Army medical team has developed microcapsules (diameters of <10 μ) containing ampicillin for topical application to wound sites.

The art is replete with patents involving the absorption or absorption of drugs on carriers. Among them are the following:

Canadian Patent No. 486,203 to Johnson & Johnson
Canadian Patent No. 503,389 to Casumano
Canadian Patent No. 823,628 to Wyant
Canadian Patent No. 547,091 to Lerner
Canadian Patent No. 588,169 to Chicopee
Canadian Patent No. 839,229 to Astra
Canadian Patent No. 1,049,407 to Pharmacia
U.S. Patent 2,381,621 patented August 7, 1945 by Wallace & Teirnan Products, Inc.
U.S. Patent No. 2,804,425 patented August 27, 1957 by American Cyanamid Company
U.S. Patent No. 3,817,702 patented June 18, 1974 by Bayer Aktiengessellschaft
U.S. Patent No. 3,987,793 patented October 26, 1976 by Ethicon Inc.
U.S. Patent No. 4,549,011 patented October 22, 1985 by Orgenics Ltd.
U.S. Patent No. 4,585,652 patented April 29, 1986 by Regents of the University of Minnesota

Enzymes, e.g. fibrinolytic proteases and deoxyribonucleases, are occasionally used to dissolve fibrous or purulent accumulations in infected wounds or burns. These enzymes are currently applied in the form of gels (e.g., carboxymethyl cellulose gel) or ointments. Such systems may suffer the same problems of allergy and time-consuming application described above. Furthermore, they do not provide mechanisms for removal of enzymic hydrolysates wich are potential irritants.

The prior art drug delivery systems (gel, cream, ointment, powder and microcapsule) suffer a practical problem : their even application or removal to and from the target site requires gentle manipulation and is too time-consuming for treatment of a large number of trauma patients in emergency cases. To overcome this problem, gauze dressings impregnated with a suspension of antibiotics (e.g., fusidic acid and Neomycin) in appropriate media (e.g., petroleum jelly and lanolin) have been developed. However, this delivery system does not control the release of drugs and thus does not solve the allergy or toxicity problems. Furthermore, the dressings impregnated with gel or liquid do not absorb the exudate, and may not provide sufficient breathability which may be desired for the treatment. US Patent 3,817,701 simply teaches that cloth may be impregnated with an antimicrobial compound which is permanently ionically bond to the cloth.

The invention is intented to provide a remedy to be above problems. It solves the problem of providing forms of dressings which can adsorb physiologically- or biologically- active compounds which can be released, in a controlled manner, to effect their biological or physiological activity. This invention thus provides a wound or burn dressing which consists of a substrate for use as a wound or burn dresssing comprising a physiologically - or biologically- active substance which is ionically bound therein, characterized in that said substrate comprises a cloth having ionic-exchange sites therein, said ionic-exchange sites being selected from the group consisting of anionic-binding sites and cationic-binding sites, whereby, upon contact with body exudate from a wound or a burn to which such dressing is applied, said active substance, in its ionic form, is released in a controlled manner by ions exchange with ions in the body exudate. This controlled release thus reduces unnecessary exposure of unwounded skin surface to the physiologically- or biologically- active agent.

The substrate which has anion-binding sites, may be a dialkylaminoalkyl cloth and is selected from the group which consists of a dimethylaminomethyl cloth, a diethylaminoethyl cloth, a diethylaminomethyl cloth, a dimethylaminoethyl cloth, a dimethylaminopropyl cloth and a diethylaminopropyl ; preferably diethylaminoethyl cloth ; or the substrate which has cation-binding sites, may be a carboxyalkyl cloth, and is selected from the group which consists of a carboxymethyl cloth, a carboxyethyl cloth and a carboxypropyl cloth ; preferably a carboxymethyl cloth.

The physiologically-active of biologically-active substance is selected from the group which consists of an antibacterial agent, an antifungal agent, an analgesic agent, a tissue healant agent, a local anesthetic agent, an antibleeding agent, an enzyme, or a vasoconstrictor or a salt form thereof.

Where the substrate is a dialkylaminoalkyl cloth, i.e. a cloth having anion-binding sites, the physiologically- or biologically- active agent is an anionic drug. Such anionic drug may be : an antibacterial, e.g. fusidic acid pseudomonic acid, ceftriaxone or nafcillin ; or an antifungal, e.g. ciclopirox, nystatin, or undecylenic acid ; or an analgesic, e.g. salicylic acid, salicylsulfuric acid or nicotinic acid ; or an antibleeding agent, e.g. adenosine diphosphate, such antibleeding agents operating by making platelets sticky, an initial step required for the stopping of bleeding. Such physiologically- or biologically- active agents may be used in the form of their salts.

Further specification of some of the above-described anionic drugs are as follows :

1. Fusidic Acid is also known as (Z)-16β-(Acetyloxy)-3α,11α-dihydroxy-29-nor-8α,9β,13α, 14β-dammara-17(20), 24-dien-21-oic acid. Its sodium salt, sodium fusidate, is also known as ZN 6.

2. Nafcillin is also known as 6-(2-Ethoxy-1-naphthamido)-3,3-dimethyl-7-oxo-4-thia-1-azabicyclo [3.2.0] heptane-2-carboxylic acid ; 6-(2-ethoxy-1-aphthamido) penicillanate. The sodium salt is also known as Naftopen and Unipen.

3. Nystatin is also known as Fungicidin.

4. Undecylenic Acid, also known as 10-Undecenoic acid.

5. Salicyclic Acid is also known as 2-Hydroxybenzoic acid.

6. Salicylsulfuric Acid is also known as 2-(Sulfooxy)- benzoic acid.

7. Nicotinic Acid is also known as 3-Pyridinecarboxylic acid.

8. Adenosine Diphosphate is also known as Adenosine 5′-(trihydrogen diphosphate).

Where the substrate is a carboxyalkyl cloth, i.e. a cloth having cation-binding sites, the physiologically- or biologically- active agent is a cationic drug. Such cationic drug may be : an antibacterial, e.g. chlorhexidine, chlorhexidine digluconate, Bacitracin, Chlortetracycline, Gentamycin, Kanamycin, Neomycin, Neomycin B, Paromomycin, Polymyxin, Polymyxin B, Streptomycin, or Tetracycline ; or an antifungal, e.g. Amphotericin B, Clotrimazole, Miconazole or Natamycin ; or tissue healants, e.g. cysteine, glycine or threonine ; or local anesthetics, e.g. Lidocaine or Pramocaine ; or enzymes, e.g. trypsin, Streptokinase, plasmin (Fibrinolysin) or Streptodornase ; or deoxyribonuclease ; or a cationic vasoconstrictor, e.g. epinephrine or serotonin. Such physiologically- or biologically- active agents may be used in the form of their salts.

Further specification of some of the above-described cationic drugs are as follows:

1. Chlorhexidine is also known as N,N″-Bis(4-chlorophenyl)-3,12-diimino-2,4,11,13-tetraazatetradecanediimidamide. Its gluconate is known as Hibiscrob.

2. Bacitracin is also known as Ayfivin.

3. Chlortetracycline is also known as 7-Chloro-4-dimethylamino-1,4,4a,5,5a,6,11,12a-octahydro-3,6,10,12,12a-pentahydroxy-6-methyl-1, 11,-dioxo-2-naphthacene carboxamide.

4. Gentamycin includes Gentamicin $C_{1a}$•, which is also known as O-3-Deoxy-4-C-methyl-3-(methylamino)-β-L-arabinopyranosyl-(1→6)-O[2,6-dramino-2,3,4,6- tetradeoxy-α-D-erythro-hexo pyranosy 1-(1→4)]-2-deoxy-D-streptamine and as gentamicin D.

Gentamicin A is also known as O-2-Amino-2-deoxy-α-D-glucopyranosyl-(1→4)-O-[3-deoxy-3-(methylamino)-α-D-xylopyranosyl-( 1→6)]-2-deoxy-D-streptamine.

The C complex sulfate is also known as Cidomycin.

5. Kanamycin includes: Kanamycin A sulfate, also known as Cantrex; Kanamycin B, is also known as NK 1006; and Kanamycin B sulfate, also known as Kanendomycin.

6. Neomycin is also known as Mycifradin. It also includes Neamine, which includes: Neomycin A, and Neomycin B, which is also known as Framycetin. Neomycin B sulfate is also known as Fraquinol.

7. Paromomycin is also known as O-2,6-Diamino-2,6-dideoxy-β-L-1-dopyranosyl-(1→3)0-β-ribofuranosyl-(1→5)-O-[2-amino-2-deoxy-α-D-glucopyranosyl-(1→4)]-2-deoxystreptamine. Its sulfate is also known as

1600 Antibiotic.

8. Polymyxin, having the structural formula

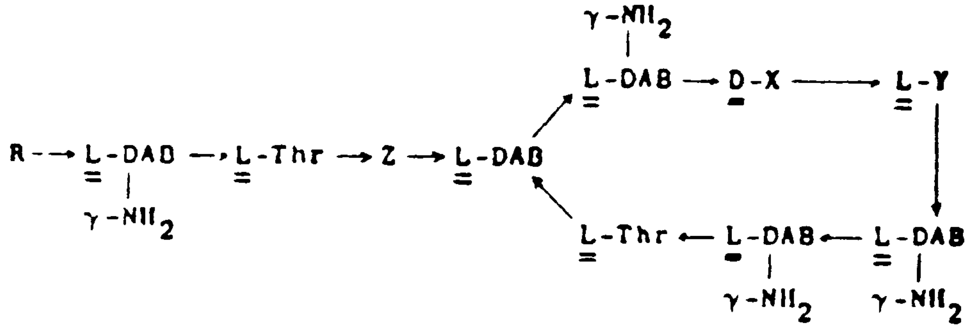

(where DAB = α,γ-diaminobutyric acid)

includes:

Polymyxin B, which is a mixture of polymyxins $B_1$ and $B_2$;

Polymyxin B sulfate, which is also known as Aerosporin;

Polymyxin $B_1$, where, in the above Formula, R = (+)-6- methyloctanoyl, X = phenylalanine, Y = leucine, and Z = L-DAB;

Polymyxin $B_1$ hydrochloride;

Polymyxin $B_2$, where, in the above Formula R = 6-methylheptanoyl, X = phenylalanine, Y = leucine, and Z = L-DAB;

Polymyxin $D_1$, where, in the above Formula R = (+)-6-methyloctanoyl, X = leucine, Y = threonine, and Z = D-serine;

Polymyxin $D_2$, where, in the above Formula R = 6-methylheptanoyl,, X = leucine, Y = threonine, and Z = D-serine; and

Polymyxin E, which is also known as Colistin.

9. Streptomycin is also known as O-2-Deoxy-2-(methylamino)-α-L-glucopyranosyl-(1→2)-O-5-deoxy-3-C-formyl-α-L-lyxofuranosyl-(1→4)-N,N′-bis(aminoiminomethyl)-D-streptamine. Its sesquisulfate is also known as streptomycin sulfate. Streptomycin B is also known as Mannosidostreptomycin.

10. Tetracycline is also known as 4-(Dimethylamino)-1,4-4a,5,5a,6,-11,12a-octahydro-3,6,10,12,12a-pentahydroxy-6-methyl-1,- 11-dioxo-2-naphthacenecarboxamide. Its phosphate complex is also known as Panmycin Phosphate. Its lauryl sulfate is known as Lauracycline.

11. Amphotericin B is also known as Fungizone.

12. Clotrimazole is also known as 1-[2-Chlorophenyl)-diphenyl-methyl]-1H-imidazole.

13. Miconazole is also known as 1-[2-(2,4-Dichlorophenyl)-2-[(2,4-dichlorophenyl)methoxy]ethyl]-1H-imidazole. Its nitrate is also known as R-14889.

14. Pimaricin is also known as Tennecetin.

15. Cysteine, Cys (IUPAC abbrev.) is also known as OL-cysteine.

16. Glycine, Gly (IUPAAC abbrev.), is also known as aminoacetic acid.

17. Threonine, Thr (IUPAC abbrev.), is also known as 2-amino-3-hydroxybutyric acid.

18. Lidocaine is also known as 2-(Diethylamino)-N-(2,6-dimethylphenyl)acetamide.

19. Pramocaine is also known as 4-[3-(4-Butoxyphenoxy)-propyl]morpholine.

20. Fibronolysin is also known as Plasmin.

21. Epinephrine is also known as 4-[1-Hydroxy-2-(methylamino)-ethyl]-1,2-benzenediol.

22. Serotonin is also known as 3-(2-aminoethyl)-1H-indol-5-ol; 5-hydroxytryptamine.

The synthesis and characterization of DEAE and CM derivatives of polysaccharides, e.g. cellulose, dextran, and starch are well-documented. The derivatives are non-toxic, non-allergenic and have been used as food additives or pharmaceuticals. Although these derivatives are provided in fiber, powder or granular forms, cloth forms of the derivatives are not commercially available. The cloth forms have definite advantages in large-scale purification and immobilization of ionic macromolecules (e.g., enzymes) because they provide easy handling (washing, transfer and removal) and faster flow in a packed bed operation. High degrees of derivatization of the cotton cloth tend to either stiffen the fabric texture or reduce the fabric strength. Furthermore, the edges of the woven fabric tend to ravel after modification. Cotton surgical dressing or gauze suffer even greater textural changes.

For topical application, pliability, softness and low linting of dressings are desired. It has been found by the present applicants that modification of nonwoven cellulose-polyester blend fabrics produces suitable ionic dressings. Unmodified, nonwoven cellulose fabrics are commercially available. For example, rayon/polyester non-woven fabric blends (known by the Trade Marks SONTARA 8801 and SONTARA 8407) may be used. They are free of chemical additives (e.g. resins), binder and finish (which would interfere with modification); are soft, pliable and low linting; and have nonravelling edges and good adsorbency. Because of the polyester part, the fabric strength is less affected by modification than cellulose fabrics. The apertured style (known by the Trade Mark SONTARA 8407) has been used as medical dressings, because of greater breathability.

Currently available methods for DEAE and CM derivation are aimed for modifying fibers or beads of polyhydroxy polymers and often require stirring and heating for relatively short periods of time. Since efficient stirring and rapid heating are difficult for modification of these fabrics in large sizes and quantities, processes have been devised by the present applicants as will be more fully described hereinafter which allow modification without stirring and at near ambient temperatures

The invention is described hereinafter as carried out with non-woven cloth, but is is to be understood this is by no means any limitative effect since the same method can be carried out with woven cloth.

For DEAE, the nonwoven rayon/polyester cloths are placed on a flat polyethylene sheet and wetted with diethylaminoethyl chloride solution (e.g., 20%) in $H_2O$ and then saturated with NaOH solution (e.g., 15%) saturated with $Na_2SO_4$. For CM, the cloths placed on a polyethylene sheet are saturated with a sodium monochloroacetate solution (e.g. 20%) in a NaOH solution (e.g. 15%).

Another polyethylene sheet is placed on top of the so-treated cloths. The sandwiched cloths are incubated at 30°C. for suitable lengths of time (normally less than 2 h). The DEAE cloths are washed with $H_2O$, 0.5 N NaOH and $H_2O$. The CM cloths are washed with $H_2O$, 0.5 N HCl and $H_2O$. Both types of cloths are air-dried. The degree of modification is controlled by time and the modified cloths must have good adsorption capacity (measured by adsorption of bovine serum albumin) and pliability and softness for contact with skin. Adsorption of fusidic acid to DEAE cloth and chlorohexidine to CM cloths has been used as model systems. The modification reaction can be carried out at any temperature between 20°C. and 50°C.

A dialkylaminoalkyl cloth other than DEAE-cloth or a carboxyalkyl cloth other than CM-cloth may be prepared in the same way as generally described above by the use of the corresponding dialkylaminoalkyl halide or by the use of a corresponding salt of a monohalocarboxylic acid.

The physiologically- or biologically- active agent can be adsorbed in the dialkylaminoalkyl or in the carboxyalkyl cloth by soaking the respective cloth in an aqueous or aqueous alcoholic solution of the active agent.

The following are Preparation Example 1 for the preparation of DEAE- and CM- cloths and Examples 2 - 4, which are embodiments of drug cloths of aspects of this invention.

Preparation Example 1, Preparation of DEAE- and CM- cloths

Nonwoven rayon/polyester blend (70/30) fabric (SONTARA 8407 from E.I. DuPont de Nemours and Co.) was used to prepare DEAE- and CM- cloths. This fabric has a unit weight of 5.1 mg per $cm^2$, and its apertured style permits good aeration when applied to skin.

For DEAE-cloth, the above-described cloth was placed on an alkali-resistant thin plastic sheet (e.g. polyethylene), uniformly moistened with 20% (w/v) diethylaminoethyl chloride hydrochloroide in $H_2O$ (0.04 ml per $cm^2$ of cloth), and then with 15% (w/v) NaOH in a saturated $Na_2SO_4$ aqueous solution (0.01 ml per $cm^2$ of cloth). A second plastic sheet was placed on the top of the cloth. This sandwiched cloth was multiplely layered between two heavy plates (glass, plastic or metal), and incubated at 30°C. for various lengths of time (e.g. 0.5h. to 2.5 h.). The cloth was then washed with water, then with 0.5 N NaOH, and water (until it had a neutral pH) and then was air-dried.

For CM-cloth, the above-described cloth, placed on a plastic sheet, was uniformly moistened with 20% (w/v sodium monochloroacetate in 15% (w/v) NaOH (0.05 ml per $cm^2$ cloth) and was then covered with a second plastic sheet. Multiple layers of the sandwiched cloth were incubated as described above for the preparation of the DEAE-cloth at 30°C. for various lengths of time (e.g. 0.5h. to 3h.). The cloth was then washed with water, then with 0.5N HCL and water (until it had a neutral pH), and then was air-dried.

The degree of cloth modification was measured by adsorption of bovine serum albumin to DEAE-cloth and egg white proteins to CM-cloth. Longer reaction times (at 30°C.) produced a cloth of higher protein adsorption, reaching a maximum adsorption of 5 mg protein per $cm^2$ of cloth (1 g protein per/g. cloth). However, overmodification generated undesirable texture (e.g. it was too hard for contact with skin or was too soft for handling). The reaction times of 1.5 h. for DEAE-cloth and 2 h. for CM-cloth were used to obtain suitable pliability and softness (with protein adsorption capacity of 2 mg/$cm^2$).

Examples of the Invention

Example 2. Adsorption of fusidic acid and chlorhexidine

Currently, gauze dressings impregnated with an ointment of fusidic acid or chlorhexidine are used to treat wounds topically. Adsorption of these antibiotics to the ionic dressings according to aspects of this invention was determined. Segments (1 cm square) of DEAE-, CM- and unmodified cloth were soaked in 1.0 ml/cloth segment of 10 mg/ml sodium fusidate or 10 mg/ml chlorhexidine digluconate for 2h. at room temperature. The segments were then washed with water.

Each segment was then soaked in 1.0 ml of porcine serum or Krebs phosphate Ringer to simulate a heavy bleeding situation (at the wound site).

The particular Krebs phosphate Ringer solution used herein had the following composition:

| | | |
|---|---|---|
| 100 Vol. | 0.154 M | NaCl |
| 4 Vol. | 0.154 M | KCl |
| 3 Vol. | 0.110 M | $CaCl_2$ |
| 1 Vol. | 0.154 M | $KH_2PO_4$ |
| 0.5 Vol. | 0.154 M | $MgSO_4$ |
| 0.45 Vol. | 0.154 M | $MgCl_2$ |

and

The amount of the respective antibiotics released were estimated by standard inhibition zone assay using an Escherichia coli ESS strain as an indicator, and are shown in Table 1.

Table 1. Antibiotics (mg) Released From Ionic Dressings (1 $cm^2$)

| Elution time (h.) | Fusidate from DEAE | | Chlorhexidine from CM | |
|---|---|---|---|---|
| | Serum | Ringer Solution | Serum | Ringer Solution |
| 0.5 | 0.25 | 0.5 | 0.1 | 0.25 |
| 1.5 | 0.5 | 0.5 | 0.25 | 0.3 |
| 24 | 0.63 | 0.75 | 1.0 | - |

Table 1 shows that the serum gradually released both antibiotics from the ionic dressings. The Ringer solution more rapidly released similar amounts of antibiotics. Since the release is due to ion exchange, macromolecular ions in the serum do not seem to be as efficient as inorganic ions in the Ringer solution. The unmodified cloth (similarly treated with the antibiotics) released negligibly small amounts of fusidate and less than a tenth the amounts of chlorhexidine released by the CM dressing.

The air-dried antibiotic dressings were stored in a light-free container at room temperature. After 4 months, the activities of the antibiotics released by the serum were determined. Both the fusidate and the chlorhexidine dressings exhibited nearly the original activity of antibiotics as described above.

Example 3. Adsorption of other drugs

Segments (1 cm square) of DEAE- and CM- cloth were soaked in 1.0 ml of 10 mg/ml of a variety of topical drugs for 2 h. at room temperature, washed with water, and then soaked in 1 ml of Krebs Ringer phosphate solution for 2 h. at room temperature. The amount of drugs released after 2 h. were estimated by ultraviolet absorption (at wavelengths between 200 to 260 nm) after diluting the extract to linear concentration vs. absorption ranges.

Table 2 (below) illustrates that all the anionic drugs tested adsorbed to the cationic DEAE-cloth, that all the cationic drugs tested adsorbed to the anionic CM-cloth, and that the adsorbed drugs can be extracted by Krebs Ringer phosphate solution. However, the results shown in Table 2 represent the amounts released with a limited volume of the Ringer solution for 2 h. and do not represent maximum adsorption of drugs.

Table 2. Drug Released Into 1 ml Krebs Ringer Solution

| Cloth | Drug | Solvent for drug adsorption | Drug released Ringer (mg) |
|---|---|---|---|
| DEAE | Sodium Fusidate | $H_2O$ | 0.52 |
| | Pseudomonic acid | Methanol | 0.33 |
| | Sodium Ceftriaxone | $H_2O$ | 0.5 |
| | Nafcilin | " | 0.36 |
| | Adenosine diphosphate | " | 0.76 |
| | Nystatin | Methanol | 0.2 |
| | Undecylenic acid | $H_2O$ | 0.2 |
| | Salicylic acid | Methanol | 0.25 |
| | Salicylsulfuric acid | $H_2O$ | 0.25 |
| | Nicotinic acid | " | 0.28 |
| CM | | | |
| | Chlorhexidine digluconate | $H_2O$ | 0.31 |
| | Bacitracin | " | 0.72 |
| | Chlortetracycline | " | 0.48 |
| | Gentamycim sulfate | " | 0.44 |
| | Kanamycin sulfate | " | 0.71 |

Table 2. (contd) Drug Released Into 1 ml Krebs Ringer Solution

| Cloth | Drug | Solvent for drug adsorption | Drug released Ringer (mg) |
|---|---|---|---|
| CM | | | |
| | Neomycin B sulfate | " | 0.24 |
| | Polymyxin B sulfate | " | 0.25 |
| | Streptomycin sulfate | " | 1.0 |
| | Tetracycline | Methanol | 0.012 |
| | Amphotericin B | " | 0.02 |
| | Clotrimazole | " | 0.02 |
| | Micronazole | " | 0.01 |
| | Cysteine | $H_2O$ | 0.12 |
| | Glycine | " | 2.0 |
| | Threonine | " | 3.0 |
| | Lidocaine | " | 0.72 |

In the above Table, the tests were conducted as follows:

A 1 cm square segment of the ionic cloth was soaked in 1 ml of 10 mg/ml of each drug dissolved in either $H_2O$ or methanol (as noted) for 2 h. at room temperature. The washed segment was soaked in 1 ml of the Ringer solution for 2 h. at room temperature. The released drugs were measured spectrometrically.

Example 4. Adsorption of trypsin and plasmin

Trypsin is capable of removing proteinaceous material from a wound. It is positively charged at a neutral pH, and therefore should be adsorbed to anionic CM-cloth. A 1 cm square segment of CM-cloth was soaked in 1 ml (per segment) of 1 mg/ml trypsin in 0.02 M sodium phosphate buffer (pH 7.0). The segment was washed with water, suspended in 1 ml of the Ringer solution, and assayed for trypsin activity. One segment of the cloth having trypsin absorbed therein showed 150 BAEE units.

Plasmin (or fibrinolysin) occurs in blood and is responsible for fibronolysis, i.e. dissolution of blood clots by the proteolytic degradation of fibrin to soluble peptides. A 1 cm square segment of DEAE-cloth was soaked in 1 ml (per segment) of 0.4 mg (1.3 U.)/ml of plasmin (plasminogen activated by streptokinase) solution. The segment was washed with water and suspended in a standard clot for activity to lyse the clot. One segment of the cloth to which plasmin was adsorbed, showed 1 U. activity.

Way In Which the Invention is Capable of Exploitation by Industry

By embodiments of the present invention, dialkylaminoalkyl, preferably diethylaminoethyl (DEAE), and carboxyalkyl, preferably carboxymethyl (CM), forms of dressings have been provided so that anionic drugs can be adsorbed onto DEAE-dressings and cationic drugs onto CM-dressing. Most of proteases are positively

charged and thus can be adsorbed to CM-dressing. Thus ionic forms of dressing have been provided to which a variety of ionic drugs (antibiotics, healants, anesthetics, etc.) as well as enzymes can be adsorbed. When applied on weeping wounds or burns, the drug will be released in controlled amounts by ion exchange with ions in the body exudate in proportion to the amount of exudate. Such a controlled release will reduce unnecessary exposure to drugs and thus to allergic reactions. The fibrinolytic enzymes can be adsorbed to the ionic dressing and may be used to dissolve fibrous or purulent accumulation and reduce inflammation. Ionic irritants generated will be adsorbed to the dressings. Use of these dressings will permit rapid and gentle application and removal of drugs and thus facilitate treatments in emergency situations, e.g. accidents, earthquakes, fires and wars.

The drug dressings of aspects of this invention can also be used to treat skin diseases, e.g., acne, or inflamation. They may also find the same applications as cosmetics, as was disclosed for the microspheres, patented by Advanced Polymer Systems, as U.S. Patent No. 4,690,825, as was described in the March 9, 1988 issue of Chemical Week.

Immobilized (adsorbed) enzymes can be used as adjuncts to antibiotic prophylaxis of surgical wounds as well as infected wounds as they show an anti-inflammatory activity. CM dressing should also adsorb cationic irritants, e.g. amines which may be generated from enzyme hydrolysis or infection. These materials can be removed with the dressing. Furthermore, CM dressing will likely adsorb a large amount of fluid and also exhibit a blood coagulating activity.

Another expected advantage of these drug systems is that the adsorbed drugs will be more stable than free drugs either in solution or suspension. Unlike dressings impregnated with drug ointments, the ionic dressings, when applied in dry forms, adsorb fluid weeping from the damaged tissues and provide better air circulation (breathability) which is often desired for the wound treatment.

It has been found that the adsorbed drugs are elutable with porcine serum and with Ringer solution and the eluted drugs have been shown to inhibit the growth of Escherichia coli. This suggests that upon application to weeping sites the dressings are capable of releasing drugs by ion exchange with ions in the blood.

## Claims

1. A wound or burn dressing which consists of a substrate for use as a wound or burn dressing comprising a physiologically -or biologically- active substance which is ionically bound thereupon, characterized in that said substrate comprises a cloth having ionic-exchange sites thereon, whereby, upon contact with body exudate from a wound or a burn to which such dressing is applied, said active substance, in its ionic form, is released in a controlled manner by ion exchange with ions in the body exudate.

2. The wound or burn dressing of claim 1, characterized in that said substrate has anion-binding sites and is a dialkylaminoalkyl cloth.

3. The wound or burn dressing of claim 2, characterized in that said dialkylaminoalkyl cloth is selected from the group which consists of a dimethylaminomethyl cloth, a diethylaminoethyl cloth, a diethylaminomethyl cloth, a dimethylaminoethyl cloth, a dimethylaminopropyl cloth and a diethylaminopropyl ; preferably diethylaminoethyl cloth.

4. The wound or burn dressing of claim 1, characterized in that said substrate has cation-binding sites and is a carboxyalkyl cloth.

5. The wound or burn dressing of claim 4, characterized in that said carboxyalkyl cloth is selected from the group which consists of a carboxymethyl cloth, a carboxyethyl cloth and a carboxypropyl cloth ; preferably a carboxymethyl cloth.

6. A wound or burn dressing of claims 1 to 5, characterized in that the said substrate is in the form of a fabric or cloth, at least a portion of which is cellulosic and has been chemically modified to convert hydroxyl groups to ionic-binding sites.

7. The wound or burn dressing of claims 1 to 6, characterized in that said physiologically- or biologically-active substance is selected from the group which consists of an antibacterial agent, an antifungal agent, an analgesic agent, a tissue healant agent, a local anesthetic agent, an antibleeding agent, an enzyme or a vasoconstrictor, or a salt form thereof.

8. The wound or burn dressing of claims 2, 3, 6 or 7, characterized in that said physiologically- or biologically- active substance is an anionic drug and is selected from the group which comprises an anionic antibacterial, especially fusidic acid, salts of fusidic acid, pseudomonic acid, ceftriaxone, or nafcillin or salts thereof ; or an anionic antifungal, especially ciclopirox, nystatin, or undecylenic acid, or salts thereof ; or an anionic analgesic, especially salicylic acid, salicylsulfuric acid or nicotinic acid, or salts thereof ; or is an anionic antibleeding agent, especially adenosine diphosphate.

9. The wound or burn dressing of claims 4, 5, 6 or 7, characterized in that said physiologically- or biologically- active substance is a cationic drug and is selected from the group which comprises an cationic antibacterial, especially chlorhexidine, bacitracin, chlortetracycline, gentamycin, kanamycin, neomycin B, paramomycin, polymyxin B, streptomycin, or tetracycline, or salts thereof ; or a cationic antifungal, especially amphotericin B, clotrimazole, miconazole or natamycin, or salts thereof ; or a cationic tissue healant, especially cysteine, glycine or threonine, or salts thereof ; a cationic local anesthetic, especially lidocaine or pramocaïne, or salts thereof ; or a cationicenzyme, especially trypsin, streptokinase, plasmin (fibrinolysin) or streptodornase, or salts thereof ; or deoxyribonuclease ; or a cationic vasoconstrictor, especially epinephrine or serotonin.

10. The wound or burn dressing of claims 1 to 9, characterized in that said substrate is a non-woven rayon/polyester cloth, preferably in the form of its apertured style.

11. A method for preparing the wound or burn dressing of claims 1 to 10, characterized it that it comprises chemically modifying by derivatizing a substrate to form ion-binding sites therein and ionically-binding thereon an ionic physiologically- or biologically- active substance.

12. The method of claim 11, characterized in that said physiologically- or biologically- active substance is bound to said modified substrate by soaking this derivatized substrate in an aqueous or aqueous alcoholic solution of said physiologically- or biologically- active substance.

13. The method of claim 11 or claim 12, characterized in that said substrate is modified to form anion-binding sites therein by means of a dialkylaminoalkyl halide, preferably a diethylaminoethyl chloride solution.

14. The method of claim 11, characterized in that said substrate is modified to form cation-binding sites therein by means of a salt of monohalocarboxylic acid, preferably a sodium monochloroacetate solution.

**Claims for the following Contracting States: GR, ES**

1. A method of preparing a wound and burn dressing which consists of a substrate in the form of a cloth comprising a physiologically -or biologically- active substance which is ionically bound thereupon, and said cloth having ionic-exchange sites thereon, whereby, upon contact with body exudate from a wound or a burn to which such dressing is applied, said active substance, in its ionic form, is released in a controlled manner by ion exchange with ions in the body exudate, characterized in that it comprises chemically modifying by derivatizing said substrate to form ion-binding sites therein and ionically-binding thereon said ionic physiologically- or biologically- active substance.

2. A method according to claim 1, characterized in that said substrate has anion-binding sites and is a dialkylaminoalkyl cloth.

3. A method according to claim 2, characterized in that said dialkylaminoalkyl cloth is selected from the group which consists of a dimethylaminomethyl cloth, a diethylaminoethyl cloth, a diethylaminomethyl cloth, a dimethylaminoethyl cloth, a dimethylaminopropyl cloth and a diethylaminopropyl ; preferably diethylaminoethyl cloth.

4. A method according to claim 1, characterized in that said substrate has cation-binding sites and is a carboxyalkyl cloth.

5. A method according to claim 4, characterized in that said carboxyalkyl cloth is selected from the group which consists of a carboxymethyl cloth, a carboxyethyl cloth and a carboxypropyl cloth ; preferably a carboxymethyl cloth.

6. A method according to claims 1 to 5, characterized in that the said substrate is in the form of a fabric or cloth, at least a portion of which is cellulosic and has been chemically modified to convert hydroxyl groups to ionic-binding sites.

7. A method according to claims 1 to 6, characterized in that said physiologically- or biologically- active substance is selected from the group which consists of an antibacterial agent, an antifungal agent, an analgesic agent, a tissue healant agent, a local anesthetic agent, an antibleeding agent, an enzyme or a vasoconstrictor, or a salt form thereof.

8. A method according to claims 2, 3, 6 or 7, characterized in that said physiologically- or biologically- active substance is an anionic drug and is selected from the group which comprises an anionic antibacterial, especially fusidic acid, salts of fusidic acid, pseudomonic acid, ceftriaxone, or nafcillin or salts thereof ; or an anionic antifungal, especially ciclopirox, nystatin, or undecylenic acid, or salts thereof ; or an anionic analgesic, especially salicylic acid, salicylsulfuric acid or nicotinic acid, or salts thereof ; or is an anionic antibleeding agent, especially adenosine diphosphate.

9. A method according to claims 4, 5, 6 or 7, characterized in that said physiologically- or biologically- active substance is a cationic drug and is selected from the group which comprises an cationic antibacterial, especially chlorhexidine, bacitracin, chlortetracycline, gentamycin, kanamycin, neomycin B, paramomycin, polmyxin B, streptomycin, or tetracycline, or salts thereof ; or a cationic antifungal, especially amphotericin B, clotrimazole,

miconazole or natamycin, or salts thereof ; or a cationic tissue healant, especially cysteine, glycine or threonine, or salts thereof ; a cationic local anesthetic, especially lidocaine or pramocaine, or salts thereof ; or a cationicenzyme, especially trypsin, streptokinase, plasmin (fibrinolysin) or streptodornase, or salts thereof ; or deoxyribonuclease ; or a cationic vasoconstrictor, especially epinephrine or serotonin.

10. A method according to claims 1 to 9, characterized in that said substrate is a non-woven rayon/polyester cloth, preferably in the form of its apertured style.

11. The method of claims 1 to 10, characterized in that said physiologically- or biologically- active substance is bound to said modified substrate by soaking this derivatized substrate in an aqueous or aqueous alcoholic solution of said physiologically- or biologically- active substance.

12. The method of claim 11, characterized in that said substrate is modified to form anion-binding sites therein by means of a dialkylaminoalkyl halide, preferably a diethylaminoethyl chloride solution.

13. The method of claim 11, characterized in that said substrate is modified to form cation-binding sites therein by means of a salt of monohalocarboxylic acid, preferably a sodium monochloroacetate solution.

## Patentansprüche

1. Wund- oder Brandwunden-Verband, der besteht aus einem Substrat für die Verwendung als Wund- oder Brandwunden-Verband, der eine physiologisch - oder biologisch - aktive Substanz enthält, die ionisch daran gebunden ist, dadurch gekennzeichnet, daß das Substrat umfaßt einen Stoff bzw. ein Gewebe mit daran befindlichen Ionenaustausch-Stellen, so daß beim Kontakt mit einem Körperexsudat aus einer Wunde oder einer Brandwunde, auf welche dieser Verband aufgelegt wird, die aktive Substanz in ihrer ionischen Form in einer kontrollierten Weise freigesetzt wird durch Ionenaustausch mit Ionen in dem Körperexsudat.

2. Wund- oder Brandwunden-Verband nach Anspruch 1, dadurch gekennzeichnet, daß das Substrat Anionen-bindende Stellen aufweist und ein Dialkylaminoalkyl-Gewebe (-Stoff) ist.

3. Wund- oder Brandwunden-Verband nach Anspruch 2, dadurch gekennzeichnet, daß das Dialkylaminoalkyl-Gewebe (-Stoff) ausgewählt wird aus der Gruppe, die besteht aus einem Dimethylaminomethyl-, Diethylaminoethyl-, Diethylaminomethyl-, Dimethylaminoethyl-, Dimethylaminopropyl- und Diethylaminopropyl-Gewebe (-Stoff), vorzugsweise Diethylaminoethyl-Gewebe (-Stoff).

4. Wund- oder Brandwunden-Verband nach Anspruch 1, dadurch gekennzeichnet, daß das Substrat Kationen-bindende Stellen aufweist und ein Carboxyalkyl-Gewebe (-Stoff) ist.

5. Wund- oder Brandwunden-Verband nach Anspruch 4, dadurch gekennzeichnet, daß das Carboxyalkyl-Gewebe (-Stoff) ausgewählt wird aus der Gruppe, die besteht aus einem Carboxymethyl-, Carboxyethyl- und Carboxypropyl-Gewebe (-Stoff), vorzugsweise Carboxymethyl-Gewebe (-Stoff).

6. Wund- oder Brandwunden-Verband nach den Ansprüchen 1 bis 5, dadurch gekennzeichnet, daß das Substrat in Form eines Gewebes oder Stoffes vorliegt, von dem mindestens ein Teil cellulosisch ist und chemisch modifiziert worden ist, um Hydroxylgruppen in Ionen-bindende Stellen umzuwandeln.

7. Wund- oder Brandwunden-Verband nach den Ansprüchen 1 bis 6, dadurch gekennzeichnet, daß die physiologischoder biologisch - aktive Substanz ausgewählt wird aus der Gruppe, die besteht aus einem antibakteriellen Agens, einem Antifungi-Agens, einem analgetischen Agens, einem Gewebeheilungs-Agens, einem lokalanästhetischen Agens, einem Antiblutungs-Agens, einem Enzym oder einem Vasokonstriktor oder einer Salzform davon.

8. Wund- oder Brandwunden-Verband nach den Ansprüchen 2, 3, 6 oder 7, dadurch gekennzeichnet, daß die physiologisch - oder biologisch - aktive Substanz ein anionisches Arzneimittel ist und ausgewählt wird aus der Gruppe, die umfaßt ein anionisches antibakterielles Mittel, insbesondere Fusidinsäure, Salze der Fusidinsäure, Pseudomonsäure, Ceftriaxon oder Nafcillin oder Salze davon; oder ein anionisches Antifungi-Mittel, insbesondere Ciclopirox, Nystatin oder Undecylensäure oder Salze davon; oder ein anionisches Analgetikum, insbesondere Salicylsäure, Salicylschwefelsäure oder Nicotinsäure oder Salze davon; oder ein anionisches Antiblutungsmittel, insbesondere Adenosindiphosphat.

9. Wund- oder Brandwunden-Verband nach den Ansprüchen 4, 5, 6 oder 7, dadurch gekennzeichnet, daß die physiologisch - oder biologisch - aktive Substanz ein kationisches Arzneimittel ist und ausgewählt wird aus der Gruppe, die umfaßt ein kationisches antibakterielles Mittel, insbesondere Chlorhexidin, Bacitracin, Chlortetracyclin, Gentamycin, Kanamycin, Neomycin B, Paramomycin, Polymyxin B, Streptomycin oder Tetracyclin oder Salze davon; oder ein kationisches Antifungi-Mittel, insbesondere Amphotericin B, Clotrimazol, Miconazol oder Natamycin oder Salze davon; oder ein kationisches Gewebe-Heilungsmittel, insbesondere Cystein, Glycin oder Threonin oder Salze davon; ein kationisches Lokal-Anästhetikum, insbesondere Lidocain oder Bramocain oder Salze davon; oder ein kationisches Enzym, insbesondere Trypsin, Streptokinase, Plasmin (Fibrinolysin) oder Streptodornase oder Salze davon; oder Deoxyribonuclease; oder ein kationischer Vasoconstriktor, insbesondere Epinephrin oder Serotonin.

10. Wund- oder Brandwunden-Verband nach einem der Ansprüche 1 bis 9, dadurch gekennzeichnet, daß das Substrat ein nicht-gewebtes Reyon/Polyester-Gewebe (-Stoff), vorzugsweise in seiner mit Löchern versehenen Form, ist.

11. Verfahren zur Herstellung des Wund- oder Brandwunden-Verbandes nach einem der Ansprüche 1 bis 10, dadurch gekennzeichnet, daß es umfaßt das chemische Modifizieren eines Substrats durch Derivatisieren unter Bildung von Ionen-bindenden Stellen darin und durch ionisches Binden einer ionischen physiologisch - oder biologisch - aktiven Substanz daran.

12. Verfahren nach Anspruch 11, dadurch gekennzeichnet, daß die physiologisch - oder biologisch - aktive Substanz an das modifizierte Substrat gebunden wird, indem man dieses derivatisierte Substrat mit einer wäßrigen oder wäßrig-alkoholischen Lösung der physiologisch - oder biologisch - aktiven Substanz imprägniert.

13. Verfahren nach Anspruch 11 oder 12, dadurch gekennzeichnet, daß das Substrat mittels einer Dialkylaminoalkylhalogenid-Lösung, vorzugsweise einer Diethylaminoethylchlorid-Lösung, modifiziert wird zur Erzeugung von Anionen-bindenden Stellen darin.

14. Verfahren nach Anspruch 11, dadurch gekennzeichnet, daß das Substrat mittels einer Monohalogencarbonsäuresalz-Lösung, vorzugsweise einer Natriummonochloracetat-Lösung, modifiziert wird zur Erzeugung von Kationen-bindenden Stellen darin.

**Patentansprüche für folgende Vertragsstaaten: ES, GR**

1. Verfahren zur Herstellung eines Wund- und Brandwunden-Verbandes, der besteht aus einem Substrat in Form eines Stoffes bzw. Gewebes, das eine physiologisch - oder biologisch - aktive Substanz enthält, die ionisch daran gebunden ist, wobei der Stoff bzw. das Gewebe Ionenaustausch-Stellen daran aufweist, so daß beim Kontakt mit Körperexsudat aus einer Wunde oder einer Brandwunde, auf welche der Verband aufgelegt wird, die aktive Substanz in ihrer ionischen Form in einer kontrollierten Weise freigesetzt wird durch Ionenaustausch mit Ionen in dem Körperexsudat, dadurch gekennzeichnet, daß es umfaßt das chemische Modifizieren des Substrats durch Derivatisieren zur Erzeugung von Ionen-bindenden Stellen darin und das ionische Binden der ionischen physiologisch - oder biologisch - aktiven Substanz daran.

2. Verfahren nach Anspruch 1, dadurch gekennzeichnet, daß das Substrat Anionen-bindende Stellen aufweist und ein Dialkylaminoalkyl-Gewebe (-Stoff) ist.

3. Verfahren nach Anspruch 2, dadurch gekennzeichnet, daß das Dialkylaminoalkyl-Gewebe (-Stoff) ausgewählt wird aus der Gruppe, die besteht aus einem Dimethylaminomethyl-, Diethylaminoethyl-, Diethylaminomethyl-, Dimethylaminoethyl-, Dimethylaminopropyl- und DiethylaminopropylGewebe (-Stoff), vorzugsweise Diethylaminoethyl-Gewebe (-Stoff).

4. Verfahren nach Anspruch 1, dadurch gekennzeichnet, daß das Substrat Kationen-bindende Stellen aufweist und ein Carboxyalkyl-Gewebe (-Stoff) ist.

5. Verfahren nach Anspruch 4, dadurch gekennzeichnet, daß das Carboxyalkyl-Gewebe (-Stoff) ausgewählt wird aus der Gruppe, die besteht aus einem Carboxymethyl-, Carboxyethyl- und Carboxypropyl-Gewebe (-Stoff), vorzugsweise Carboxymethyl-Gewebe (-Stoff).

6. Verfahren nach den Ansprüchen 1 bis 5, dadurch gekennzeichnet, daß das Substrat in Form eines Gewebes oder Stoffes vorliegt, der mindestens zum Teil cellulosisch ist und chemisch modifiziert worden ist, um die Hydroxylgruppen in Ionen-bindende Stellen umzuwandeln.

7. Verfahren nach den Ansprüche 1 bis 6, dadurch gekennzeichnet, daß die physiologisch - oder biologisch - aktive Substanz ausgewählt wird aus der Gruppe, die besteht aus einem antibakteriellen Agens, einem Antifungi-Agens, einem analgetischen Agens, einem Gewebeheilungs-Agens, einem lokalanästhetischen Agens, einem Antiblutungs-Agens, einem Enzym oder einem Vasokonstriktor oder einer Salzform davon.

8. Verfahren nach den Ansprüchen 2, 3, 6 oder 7, dadurch gekennzeichnet, daß die physiologisch - oder biologischaktive Substanz ein anionisches Arzneimittel ist und ausgewählt wird aus der Gruppe, die umfaßt ein anionisches antibakterielles Mittel, insbesondere Fusidinsäure, Salze der Fusidinsäure, Pseudomonsäure, Ceftriaxon oder Nafcillin oder Salze davon; oder ein anionisches Antifungi-Mittel, insbesondere Ciclopirox, Nystatin oder Undecylensäure oder Salze davon; oder ein anionisches Analgetikum, insbesondere Salicylsäure, Salicylschwefelsäure oder Nicotinsäure oder Salze davon; oder ein anionisches Antiblutungs-Mittel, insbesondere Adenosindiphospaht.

9. Verfahren nach den Ansprüchen 4, 5, 6 oder 7, dadurch gekennzeichnet, daß die physiologisch - oder biologischaktive Substanz ein kationisches Arzneimittel ist und ausgewählt wird aus der Gruppe, die umfaßt ein kationisches antibakterielles Mittel, insbesondere Chlorhexidin, Bacitracin, Chlortetracyclin, Gentamycin, Kanamycin, Neomycin B, Paramomycin, Polymyxin B, Streptomycin oder Tetracyclin oder Salze davon; oder ein kationisches Antifungi-Mittel, insbesondere Amphotericin B, Clotrimazol, Miconazol oder Natamycin oder

Salze davon; oder ein kationisches GewebeHeilungsmittel, insbesondere Cystein, Glycin oder Threonin oder Salze davon; ein kationisches Lokalanästhetikum, insbesondere Lidocain oder Bramocain oder Salze davon; oder ein kationisches Enzym, insbesondere Trypsin, Streptokinase, Plasmin (Fibrinolysin) oder Streptodornase oder Salze davon; oder Deoxyribonuclease; oder ein kationischer Vasokonstrictor, insbesondere Epinephrin oder Serotonin.

10. Verfahren nach den Ansprüchen 1 bis 9, dadurch gekennzeichnet, daß das Substrat ein nicht-gewebtes Reyon/Polyester-Gewebe (-Stoff), vorzugsweise in seiner mit Löchern versehenen Form, ist.

11. Verfahren nach den Ansprüchen 1 bis 10, dadurch gekennzeichnet, daß die physiologisch - oder biologischaktive Substanz an das modifizierte Substrat gebunden wird durch Imprägnieren dieses derivatisierten Substrats mit einer wäßrigen oder wäßrig-alkoholischen Lösung der physiologisch - oder biologisch - aktiven Substanz.

12. Verfahren nach Anspruch 11, dadurch gekennzeichnet, daß das Substrat mittels einer Dialkylaminohalogenid-Lösung, vorzugsweise einer Diethylaminoethylchlorid-Lösung, modifiziert wird zur Erzeugung von Anionen-bindenden Stellen darin.

13. Verfahren nach Anspruch 11, dadurch gekennzeichnet, daß das Substrat mittels einer Monohalogencarbonsäuresalz-Lösung, vorzugsweise einer Natriummonochloracetat-Lösung, modifiziert wird zur Erzeugung von Kationen-bindenden Stellen darin.

## Revendications

1. Pansement pour brûlure ou plaie qui consiste en un substrat utile en tant que pansement pour brûlure ou plaie comprenant une substance active du point de vue physiologique ou biologique qui est liée ioniquement à ce substrat, caractérisé en ce que ce substrat comprend un tissu portant des sites d'échange ionique, si bien qu'au contact d'un exsudat corporel provenant de la brûlure ou de la plaie sur laquelle est appliquée ce pansement, cette substance active, sous sa forme ionique, est libérée de façon contrôlée par échange d'ions avec des ions de l'exsudat du corps.

2. Pansement pour brûlure ou plaie suivant la revendication 1, caractérisé en ce que ce substrat présente des sites pour fixer des anions et est un tissu au dialkylaminoalkyle.

3. Pansement pour brûlure ou plaie suivant la revendication 2, caractérisé en ce que ce tissu au dialkylaminoalkyle est choisi parmi un tissu au diméthylaminométhyle, un tissu au diéthylamoinoéthyle, un tissu au diéthylaminométhyle, un tissu au diméthylaminoéthyle, un tissu au diméthylaminopropyle et un tissu au diéthylaminopropyle et de préférence, un tissu au diéthylaminoéthyle.

4. Pansement pour brûlure ou plaie suivant la revendication 1, caractérisé en ce que ce substrat présente des sites pour fixer des cations et est un tissu au carboxyalkyle.

5. Pansement pour brûlure ou plaie suivant la revendication 4, caractérisé en ce que ce tissu au carboxyalkyle est choisi dans le groupe qui consiste en tissu au carboxyméthyle, un tissu au carboxyéthyle et un tissu au carboxypropyle et de préférence, un tissu au carboxyméthyle.

6. Pansement pour brûlure ou plaie suivant les revendications 1 à 5, caractérisé en ce que ce substrat est sous la forme d'un tissu ou d'une étoffe dont une portion au moins est cellulosique et a été modifié chimiquement pour convertir des groupes hydroxy en sites de liaison ionique.

7. Pansement pour brûlure ou plaie suivant les revendications 1 à 6, caractérisé en ce que cette substance active du point de vue physiologique ou biologique est choisie dans le groupe comprenant un agent antibactérien, un agent antifongique, un agent analgésique, un agent cicacrisant les tissus, un agent anesthésique local, un agent hémostatique, une enzyme ou un vasoconstricteur ou une forme saline de ceux-ci.

8. Pansement pour brûlure ou plaie suivant les revendications 2, 3, 6 ou 7, caractérisé en ce que cette substance active du point de vue physiologique ou biologique est un médicament anionique et qu'elle est choisie dans le groupe qui comprend un agent antibactérien anionique, en particulier l'acide fusidique, les sels d'acide fusidique, l'acide pseudomonique, la céftriaxone ou la nafcilline, ou leurs sels; ou un agent antifongique anionique, en particulier le ciclopirox, la nystatine ou l'acide undécylénique, ou leurs sels; ou un agent analgésique anionique, en particulier l'acide salicylique, l'acide salicylsulfurique ou l'acide nicontinique ou leurs sels; ou un agent hémostatique anionique, en particulier l'adénosine diphosphate.

9. Pansement pour brûlure ou plaie suivant les revendications 4, 5, 6 ou 7, caractérisé en ce que cette substance active du point de vue physiologique ou biologique est un médicament cationique et qu'elle est choisie dans le groupe qui comprend un agent antibactérien cationique, en particulier la chlorhexidine, la bacitracine, la chlortétracyline, la gentamycine, la kanamycine, la néomycine B, la paramomycine, la polymyxine B, la streptomycine ou la tétracycline, ou leurs sels; ou un agent antifongique cationique, en particulier l'amphotéricine B, le clotrimazole, le miconazole ou la natamycine, ou leurs sels; ou un agent cationique cicatrisant

les tissus, en particulier la cystéine, la glycine ou la thréonine, ou leurs sels; un agent anesthésique local cationique, en particulier la lidocaïne ou la pramocaïne, ou leurs sels; ou une enzyme cationique, en particulier la trypsine, la streptokinase, la plasmine (fibrinolysine) ou la streptodornase, ou leurs sels; ou la désoxyribonucléase; ou un vasoconstricteur cationique, en particulier l'épinéphrine ou la sérotonine.

10. Pansement pour brûlure ou plaie suivant les revendications 1 à 9, caractérisé en ce que ce substrat est un tissu de rayonne/polyester non tissé, de préférence sous la forme de son modèle perforé.

11. Procédé pour préparer le pansement pour brûlure ou plaie suivant les revendications 1 à 10, caractérisé en ce qu'il comprend la modification chimique par formation d'un dérivé d'un substrat pour y former des sites pour fixer des ions et la liaison par voie ionique sur ceux-ci d'une substance active du point de vue physiologique ou biologique.

12. Procédé suivant la revendication 11, caractérisé en ce que cette substance active au point de vue physiologique ou biologique est liée à ce substrat modifié par imprégnation de ce substrat dérivé dans une solution aqueuse ou alcoolo-aqueuse de cette substance active du point de vue physiologique ou biologique.

13. Procédé suivant la revendication 11 ou la revendication 12, caractérisé en ce que ce substrat est modifié pour former des sites pour fixer des anions dans celui-ci au moyen d'un halogènure de dialkylaminoalkyle, de préférence une solution de chlorure de diéthylaminoéthyle.

14. Procédé suivant la revendication 11, caractérisé en ce que ce substrat est modifié pour former des sites pour fixer des cations dans celui-ci au moyen d'un sel d'acide monohalocarboxylique, de préférence une solution de monochloroacétate de sodium.

**Revendications pour les Etats contractants suivants: GR, ES**

1. Procédé pour préparer un pansement pour brûlure ou plaie qui consiste en un substrat sous la forme d'un tissu comprenant une substance active au point de vue physiologique ou biologique qui est liée ioniquement à celui-ci et ce tissu portant des sites d'échange ionique, si bien qu'au contact d'un exsudat corporel provenant de la brûlure ou de la plaie sur laquelle est appliqué ce pansement, cette substance active, sous sa forme ionique, est libérée de façon contrôlée par échange d'ions avec des ions de l'exsudat du corps, caractérisé en ce qu'il comprend la modification chimique par formation d'un dérivé de ce substrat pour y former des sites pour fixer des ions et la liaison par voie ionique sur ceux-ci d'une substance active du point de vue physiologique ou biologique.

2. Procédé suivant la revendication 1, caractérisé en ce que ce substrat présente des sites pour fixer des anions et est un tissu au dialkylaminoalkyle.

3. Procédé suivant la revendication 2, caractérisé en ce que ce tissu au dialkyl-aminoalkyle est choisi parmi un tissu au diméthylaminométhyle, un tissu au diéthylamoinoéthyle, un tissu au diéthylaminoéthyle, un tissu au diméthylaminoéthyle, un tissu au diméthylaminopropyle et un tissu au diéthylaminopropyle et de préférence, un tissu au diéthylaminoéthyle.

4. Procédé suivant la revendication 1, caractérisé en ce que ce substrat présente des sites pour fixer des cations et est un tissu au carboxyalkyle.

5. Procédé suivant la revendication 4, caractérisé en ce que ce tissu au carboxyalkyle est choisi dans le groupe qui consiste en tissu au carboxyméthyle, un tissu au carboxyéthyle et un tissu au carboxypropyle et de préférence, un tissu au carboxyméthyle.

6. Procédé suivant les revendications 1 à 5, caractérisé en ce que ce substrat est sous la forme d'un tissu ou d'une étoffe dont une portion au moins est cellulosique et a été modifiée chimiquement pour convertir des groupes hydroxy en sites de liaison ionique.

7. Procédé suivant les revendications 1 à 6, caractérisé en ce que cette substance active au point de vue physiologique ou biologique est choisie dans le groupe comprenant un agent antibactérien, un agent antifongique, un agent analgésique, un agent cicacrisant les tissus, un agent anesthésique local, un agent hémostatique, une enzyme ou un vasoconstricteur ou une forme saline de ceux-ci.

8. Procédé suivant les revendications 2, 3, 6 ou 7, caractérisé en ce que cette substance active au point de vue physiologique ou biologique est un médicament anionique et qu'elle est choisie dans le groupe qui comprend un agent antibactérien anionique, en particulier l'acide fusidique, les sels d'acide fusidique, l'acide pseudomonique, la céftriaxone ou la nafcilline, ou leurs sels; ou un agent antifongique anionique, en particulier le ciclopirox, la nystatine ou l'acide undécylénique, ou leurs sels; ou un agent analgésique anionique, en particulier l'acide salicylique, l'acide salicysulfurique ou l'acide nicotinique ou leurs sels; ou un agent hémostatique anionique, en particulier l'adénosine diphosphate.

9. Procédé suivant les revendications 4, 5, 6 ou 7, caractérisé en ce que cette substance active du point de vue physiologique ou biologique est un médicament cationique et qu'elle est choisie dans le groupe qui comprend un agent antibactérien cationique, en particulier la chlohexidine, la bacitracine, la chlortétracycline,

la gentamycine, la kanamycine, la néomycine B, la paramomycine, la polymyxine B, la streptomycine ou la tétracycline, ou leurs sels; ou un agent antifongique cationique, en particulier l'amphotéricine B, le clotrimazole, le miconazole ou la natamycine, ou leurs sels; ou un agent cationique cicatrisant les tissus, en particulier la cystéine, la glycine ou la thréonine, ou leurs sels; un agent anesthésique local cationique, en particulier la lidocaïne ou la pramocaïne, ou leurs sels; ou une enzyme cationique, en particulier la trypsine, la streptokinase, la plasmine (fibrinolysine) ou la streptodornase, ou leurs sels; ou la désoxyribonucléase; ou un vasoconstricteur cationique, en particulier l'éphinéphrine ou la sérotonine.

10. Procédé suivant les revendications 1 à 9, caractérisé en ce que ce substrat est un tissu de rayonne/polyester non tissé, de préférence sous la forme de son modèle perforé.

11. Procédé suivant les revendications 1 à 10, caractérisé en ce que cette substance active du point de vue physiologique ou biologique est liée à ce substrat modifié par imprégnation de ce substrat dérivé dans une solution aqueuse ou alcoolo-aqueuse de cette substance active du point de vue physiologique ou biologique.

12. Procédé suivant la revendication 11, caractérisé en ce que ce substrat est modifié pour former des sites pour fixer des anions dans celui-ci au moyen d'un halogènure de dialkylaminoalkyle de préférence une solution de chlorure de diéthylaminoéthyle.

13. Procédé suivant la revendication 11, caractérisé en ce que ce substrat est modifié pour former des sites pour fixer des cations dans celui-ci au moyen d'un sel d'acide monohalocarboxylique, de préférence une solution de monochloroacétate de sodium.